(19) Europäisches Patentamt / European Patent Office / Office européen des brevets

(11) EP 2 734 944 B1

(12) **EUROPEAN PATENT SPECIFICATION**

(45) Date of publication and mention
of the grant of the patent:
**23.03.2016 Bulletin 2016/12**

(51) Int Cl.:
***G06F 19/00*** *(2011.01)*

(21) Application number: **12712097.0**

(22) Date of filing: **27.03.2012**

(86) International application number:
**PCT/EP2012/055441**

(87) International publication number:
**WO 2013/010685 (24.01.2013 Gazette 2013/04)**

(54) **A METHOD AND APPARATUS FOR SELECTING A PRODUCT**

VERFAHREN UND VORRICHTUNG ZUR AUSWAHL EINES PRODUKTS

PROCÉDÉ ET APPAREIL DE SÉLECTION D'UN PRODUIT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **19.07.2011 GB 201112421**

(43) Date of publication of application:
**28.05.2014 Bulletin 2014/22**

(73) Proprietor: **Gene Onyx Ltd
Road Town, Tortola (VG)**

(72) Inventors:
• **TOUMAZOU, Christofer
London W6 0UH (GB)**
• **SIM, Calvin
Hong Kong (CN)**

(74) Representative: **Lind, Robert
Marks & Clerk LLP
Fletcher House
Heatley Road
The Oxford Science Park
Oxford OX4 4GE (GB)**

(56) References cited:
WO-A1-2010/062006    DE-A1- 10 056 203
US-A- 5 622 692    US-A1- 2003 124 553
US-A1- 2007 166 204    US-A1- 2009 094 059
US-A1- 2010 086 501    US-A1- 2010 145 725
US-A1- 2010 255 595    US-A1- 2011 082 867
US-A1- 2011 086 352    US-B2- 6 782 307

**Description**

Technical Field

The present invention relates to a method and apparatus for selecting a product.

Background

The cosmetic products market is large and diverse with numerous brands, product types and wide ranges of similar products. A recent market research survey has found that in 2010 consumers spent nearly US$53 billion on cosmetics and beauty products. The array of choices of available cosmetic products may be bewildering and an individual's approach to selecting a product could typically be based on any combination of the following selection criteria: brand, price, packaging aesthetics, indicated usage, prior experience where available and also marketing input from the retail assistant. Often, where there is no prior experience with the product, there is little, if any, guidance tailored to an individual that could be relied upon to indicate the efficacy and allergenic potential of the cosmetic product to that individual.

Consequently, a trial and error method is often used by consumers to assess and purchase cosmetic products. When a purchased product turns out to be unsuitable or ineffective, the consumer will discontinue using the product, and often will also discard it, leading to wastage and consumer frustration. In some more severe cases, and particularly for people with sensitive skin, adverse allergic reactions may occur leading to unsightly reddening, swelling and/or itching, and in extreme cases, may be life threatening.

Currently, manufacturers, marketers and/or vendors of cosmetic products are attempting to address this problem through the use of scientific methods of investigation and analysis of a potential buyer's skin condition. When this is performed "on-site" (e.g. in the shop with the potential buyer present), the results and conclusions are used to offer recommendations on appropriate products and/or remedies. However, on-site investigations and analysis currently only address the physiological expression or symptom of traits of the individual and does not shed any insight on the underlying genetic cause of the predisposition or condition.

"Off-site" testing (e.g. away from the shop, when the potential buyer is no longer present) allows for investigations based on genotyping of the individual and these tests are now available commercially. However, the need to send their genetic material to a remote site can increase a worried consumer's concerns on the storage and tracking of individual genetic material. Moreover, given that the successful sale of new cosmetic products is often driven by meeting a mixture of human needs and desires in a timely fashion, it is important that this process of product guidance is performed within a short timeframe and while the potential customer's interest is sustained. In discerning and accurately responding to a customer's needs, having a sales assistant who is able to guide and respond in a personable, face-to-face manner also significantly increases the likelihood of achieving customer satisfaction. In this regard, existing commercial products that require genetic tests to be performed off-site are unable to have or sustain a personal engagement with the customer right through the entire process.

GB 2389424 discloses a rapid genotyping technology which is capable of performing single nucleotide polymorphisms (SNPs) genotyping through electro-chemical means in the order of minutes rather than days with traditional optical PCR approaches.

Summary

According to a first aspect of the invention there is provided a method of using a point-of-sale terminal to select a product suited to an individual, the method being carried out at retail premises and comprising:

- accessing a memory to identify a product category from a predefined set of product categories, each product category being associated with a set of products and,
- on the basis of said selected product category, select a genetic testing cartridge type from a set of available cartridge types, each cartridge type being configured to perform a genotype profiling test on a polynucleotide sample;
- collecting a polynucleotide sample from the individual, wherein said step of collecting a polynucleotide sample from an individual is performed by the individual or a point-of-sale representative, the individual or point-of-sale representative applying the collected sample to a genetic testing cartridge of the selected type;
- performing a genotype profiling test on said polynucleotide sample using a genetic testing cartridge of the selected genetic testing cartridge type; and
- obtaining, via an interface coupling the point-of-sale terminal to the genetic testing cartridge, the test result and using the result of said test to select a product from within the identified product category.

The step of identifying a product category comprises identifying a product category to a point-of-sale terminal using a graphical user interface of the terminal, the point-of-sale terminal performing the steps of selecting a genetic testing cartridge type and using the results of said test to select a product from within the identified product category, the selection being displayed to a terminal user via said graphical user interface.

In a first embodiment of the invention, the step of using the result of said test to select a product comprises using the result of said test to select one or more metabolisable compounds from a group of known metabolisable compounds, and then selecting a product from the identified product category that contains at least one of the one or more selected metabolisable compounds.

The one or more metabolisable compounds may be selected by determining the efficacy of the known metabolisable compounds with respect to the metabolism pathway of the individual, and selecting the one or more with the highest efficacy.

The polynucleotide sample may contain DNA, RNA or a combination of both.

Said genotype profiling test may comprise one or more single-nucleotide polymorphism tests.

Said product may be one of: a skin care product, a cosmetic, a cosmeceutical, a nutricosmetic, nutritional supplement, a food group, and an agricultural product.

Said step of using the result of said test to select a product from within the identified product category may comprise performing a lookup in a product database for the identified product category, using as lookup key the test result.

Said step of identifying a product category may comprise identifying a product category to a point of sale terminal using a graphical user interface of the terminal, the point of sale terminal performing the steps of selecting a genetic testing cartridge type and using the results of said test to select a product from within the identified product category, the selection being displayed to a terminal user via said graphical user interface.

The method may comprise deleting all records of the test result from the point of sale terminal following selection of a product.

The method may comprise receiving a questionnaire response from said individual, and using said response in the selection of a genetic testing cartridge type and/or in the selection of a product.

Said genetic testing cartridge of the selected genetic testing cartridge type may be configured to determine the efficacy of the associated product set.

Said step of selecting a product category may comprise:

collecting a polynucleotide sample from the individual;

using a genetic testing cartridge to perform a genotype profiling test and using the result of the test to identify a predisposition of the individual to each of one or more conditions; and

for a given one or combination of the identified predispositions, choosing a product category that can potentially treat the associated condition(s).

The method may comprise using genetic information and customer information, obtained for the individual, in the identification of the condition predispositions and/or the choosing of the product category.

The method may comprise the genetic information of an individual is obtained using a cheek swab, a saliva sample or a blood test.

The method may further comprise that the customer information of the individual is obtained via a questionnaire.

According to a second aspect of the invention there is provided a point of sale terminal comprising:

access to memory that may be local or remote to the terminal, in which memory there is stored a first database containing a set of product categories and a set of genetic testing cartridge types mapped to the product categories, and a second database containing, for each product category, a set of available products and a set of genotype profiling outcomes mapped to the available products;

an interface adapted to couple the point of sale terminal to a genetic testing cartridge in order to receive test results from the cartridge;

a graphical user interface adapted to receive from a terminal user an identification of a product category and for displaying to the user a selected product; and a processing entity adapted to use the received identification of a product category to lookup in said first database to select a genetic testing cartridge type, for receiving a test result via said interface, use the received test result as a lookup in said second database to select a product, and cause the graphical user interface to display the selected product to a user.

Brief Description of the Drawings

Figure 1 shows a typical service cycle of a customer according to an embodiment of the current invention;

Figure 2 shows a flow diagram outlining the steps of a method to provide guidance to increase the likelihood of a particular product being suitable for an individual;
Figure 3 shows a graphical depiction of a Recommendations Engine interfacing with a Test Cartridge;
Figure 4 shows a block diagram of a recommendations engine according to an embodiment of the invention; and
Figure 5 shows a block diagram of a Test Cartridge for use in rapid genotyping.

Detailed Description

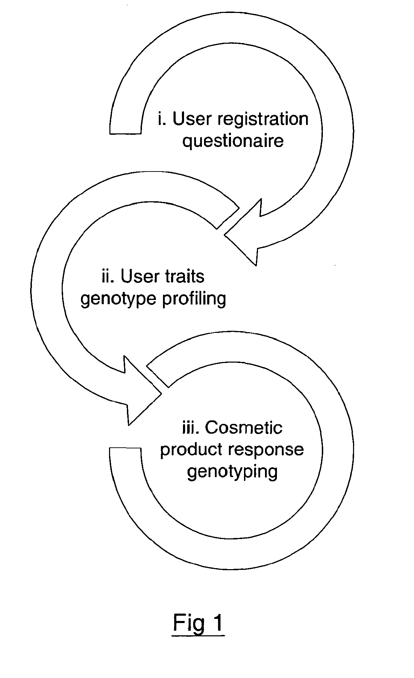

Figure 1 shows the typical service cycle of a customer according to an embodiment of the current invention. The main components of the cycle are:

i. user registration in which the customer's information is captured through a user questionnaire and can be uploaded to the user database in a central database, such as on an Internet cloud server;
ii. initial genotype profiling to determine the key traits of the customer, such as the customer's propensity to aging, inflammation, etc; and
iii. the customer's response to a set of cosmetic products is predicted through the further capture and analysis of the customer's genotype by profiling the customer against a set of known SNP mutations that characterise the customer's response to specific chemical compounds that exist in the cosmetic product in terms of efficacy and allergenic response. This final step may be repeated each time the customer would like to evaluate the suitability of a recommended cosmetic product.

Taking cosmetic products that are targeted for improving a user's skin as an example, in terms of skin health, there are a number of key pillars that support healthy skin.
Factors that affect healthy skin may generally be grouped according to these pillars. The key pillars are:

1. neutralisation of reactive oxygen species;
2. epidermal immune defense;
3. collagen reinforcement;
4. moisture retention/hydration;
5. nourishment and oxygenation; and
6. detoxification.

For each pillar, the factors that effect skin health may include both phenotypical (e.g. environmental) as well as genotypical (i.e. genetic) contributions.
The expressed factors responsible for the phenotypical characteristic of an individual user for a particular pillar of skin health could be determined from responses to a customer questionnaire. In one instance, the questionnaire could be presented as a collection of statements to which the respondent has to rate on a scale, such as from 0 to 5, to what extent they would agree with each statement. The statements may be carefully and deliberately constructed in consultation with expert advice to provide an accurate assessment of an individual's skin health in relation to one or more key pillars.
To ensure consistency, the impact of each of the statements towards each pillar of skin health may be quantified and collated in a matrix, as illustrated in Table 1.
It is then be possible to mathematically model and derive a quantitative assessment for each pillar of an individual's skin health based on the individual's questionnaire responses. The individual's skin health may then be characterised numerically as $\{Q_{p1}, Q_{p2}, ..., Q_{p6}, Q_{a1}\}$, where $Q_{pn}$ and $Q_{a1}$ are the aggregate scores for each pillar of skin health and allergenic response respectively. A score of zero would indicate ideal healthy skin while any score greater than zero would indicate skin health deficiency.
The next stage of the service cycle is to build up a genotypical profile of the individual similar to the phenotypical profile generated using the customer questionnaire. However, instead of a score based on the individual's responses to specific statements, the score is determined by the genotype of the individual with respect to a set of key genes that regulate skin health. For each of the key genes to be included in the genotyping procedure, its impact to each of the key pillars of skin health is preanalysed and tabulated within a matrix. For example, the gene MMP1 may have strong impact on collagen reinforcement, some impact on nourishment and oxygenation but minimal impact on skin hydration. For other key genes, their impact on the different pillars will of course be different. The differences are quantitatively related using a matrix. An example of the matrix is shown in Table 2.
The values in the matrix may be stored on the Internet at a cloud server and would only be updated infrequently so as to maximise the level of consistency of the data.
The individual's profile based on his or her genotype for each pillar of skin health may be calculated based on the pre-computed matrix and his/her genotype (including heterozygosity). In this manner, the individual's skin for each of key pillars based on his or her genotype may be numerically characterised as $\{G_{p1}, G_{p2}, ..., G_{p6}, G_{a1}\}$, where $G_{pn}$ and $G_{a1}$ are the aggregate scores for each pillar of skin health and allergenic response respectively. A score of zero would indicate ideal healthy skin while any score greater than zero would indicate a level of skin health deficiency.

In view of the number of cosmetic products that would match each selection of SNPs, there are a number of ways in which different brands and retailers of cosmetic products will be able to test their customer using their genetic make-up to determine their product's suitability. The customer's genetic information is extracted, for example by a cheek swab, saliva sample, blood test or similar, and then analysed and mapped to the expected phenotype characteristics that those SNPs code for. These steps are carried out in-situ, such that none of the customer's genetic information is transmitted or stored. Rather than providing the genetic information directly to the customer, the outcome of the test is an analysis of the suitability of the customer to a recommended cosmetic product. For example, the customer may be told that he or she has a predisposition for dry skin, and so should consider using skin creams. Consequently, the same set of genetic information may be repeatedly tested so as to ascertain the customer's suitability to different cosmetic brands and products within an available set of skin creams.

It is envisioned that the primary focus of this system is in matching the efficacy and allergenic response of specific skin-care/cosmetic products that are targeted towards improving/augmenting skin health to specific individuals. However, it could equally be possible that such a system is used purely for identifying possible allergenic risks to specific individuals for cosmetic products solely for aesthetic functionality, or for cosmeceuticals, nutricosmetics, nutritional supplements, food groups and/or agricultural products.

Most cosmetic/skin-care products comprise one or more active compounds which are responsible for the target and/or marketed function of the product, as well as supporting bulk compounds. The active compound(s) in a cosmetic product typically target one or more of the pillars of skin health described above in terms of augmenting the intrinsic capabilities of the individual. Information of the use of such a compound as the active ingredient in a dermatological product can be obtained empirically or is available from the literature provided with the cosmetic product. Furthermore, for each compound (both active and bulk), the relative risk of inflammation due to that compound can be quantified with results obtained either empirically through laboratory testing or existing published results.

Consequently, a matrix estimating the quantitative effect of each compound/reagent to each of the pillars of skin health and their associated risk of inflammation can be collated, an example of which is shown in Table 3.

In the example in Table 3, compounds NIA-114, Azathioprine, aaa and bbb are active compounds which would affect skin health while compounds ccc and ddd would be bulk compounds which have little effect on skin health but may still provoke an allergenic response. With such a matrix, it is possible to mathematically model and derive a formula that can quantitatively estimate, and provide a corresponding numerical score, for the efficacy of any skin-care/cosmetic product towards achieving one or more pillars of skin-care health as an aggregate of its compounds, so long as all the constituent compounds and their relative concentration to a standardised volume is known. Similarly, it would be possible to model the product's allergenic response impact for any skin-care/cosmetic product given similar criteria and inputs.

In one instance, the aggregate effect of the product towards p1 may be modelled by:

$$E_{pn} = \sum \gamma_{r_n} p_{r_n} \tag{1}$$

and the aggregate allergenic response may be modelled by:

$$\alpha = \sum \gamma_{r_n} a_{r_n} \tag{2}$$

For example, a skin-care product targeted at neutralising free radicals (p1) may comprise compounds r1, r2, r5 and r6 with standardised concentrations of 0.05, 0.1, 0.4 and 0.7 respectively. The impact of the product towards p1 would therefore be estimated as:

$$E_{p1} = 0.05 \times 0.8 + 0.1 \times 0.3 = 0.07$$

Similarly, the allergenic response of the product would be estimated as:

$$\alpha_{a1} = 0.05 \times 0.1 + 0.1 \times 0.4 + 0.4 \times 0.1 + 0.7 \times 0.2 = 0.225$$

For each product, it is possible to quantitatively characterise its effectiveness towards each pillar of skin health with $\{E_{p1}, E_{p2}, ..., E_{p6}, E_{a1}\}$, where $E_{pn}$ and $E_{a1}$ are the aggregate scores for each pillar of skin health and allergenic response respectively. A score of zero would indicate minimum effectiveness to a particular deficiency while any score greater than zero would quantitatively indicate the level of effectiveness.

Using such an approach, each skin-care product within the recommendation system can be scored and ranked in order for each pillar of skin-health and/or potential allergenic risk. Given the user's skin health characteristic has been previously determined through the user questionnaire and genotype profiling, the list of most relevant skin-care or cosmetic product(s) that is specific to the individual may be determined by minimising the following expression:

$$Q_{pn} + G_{pn} - E_{pn} \qquad (3)$$

In other words, the products that are most relevant to a specific individual are products where the user's characteristics as quantified by $Q_{pn} + G_{pn}$ are best matched by the level of effectiveness for a particular pillar of skin health, $E_{pn}$, of that product.

Ideally, a particular product would work as indicated and provide the desired effect. However, due to the occurrence of SNP mutations that may impact the action/metabolism of the active ingredient(s) in the mechanism pathway, the efficacy of those ingredients may be diminished either completely or partially. It is the objective of the third stage of the service cycle to use genotyping means to evaluate the efficacy of a product to a specific individual and also predict the risk of an allergenic response to the product. The regulation of the effect of a particular product due to mutations in the genotype may be characterised by an efficacy coefficient, $\gamma_{pn}$, such that the actual compensatory effect of the product for a pillar of skin health would be $\gamma_{pn}E_{pn}$. Hence, in reality, the expression (3) above should be modified to:

$$Q_{pn} + G_{pn} - \gamma_{pn} E_{pn} \qquad (4)$$

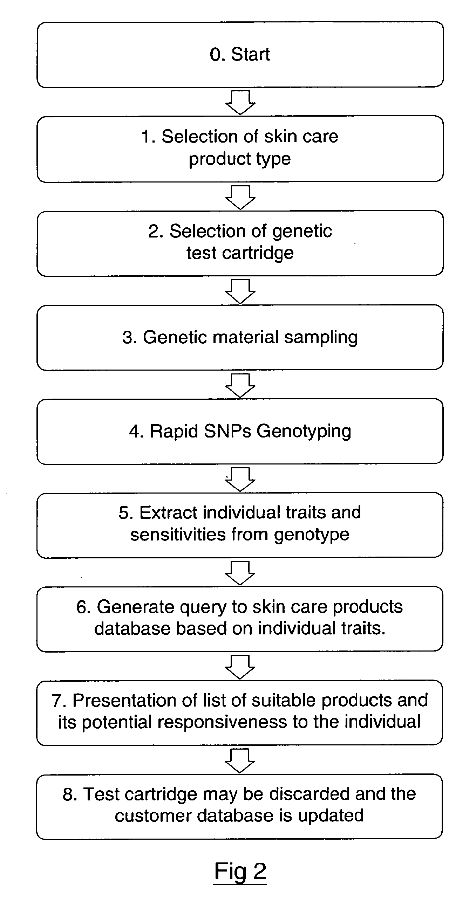

The genotyping performed in the third stage of the service life-cycle is focused on determining the value of $\gamma_{pn}$ for each of the key pillars of skin health. Due to the effect of mutation on the efficacy of the active ingredients in its effectiveness, skin-care products that are expected to work well for a particular skin-health objective for a particular user with a known skin-health characteristic, may still not work as well in reality. By determining the impact of such mutations on the efficacy of a range of potentially suitable products, the suitability for that range of products may be more accurately assessed. Figure 2 shows a flow diagram that describes a method to provide objective guidance to this process in order to significantly increase the likelihood of a particular skin-care product being suitable for an individual. This is achieved by ascertaining specific traits coded within the genetic material of the individual and matching them to a database of cosmetic products and their constituent ingredients, from which a substantially smaller subset of suitable cosmetic products is presented.

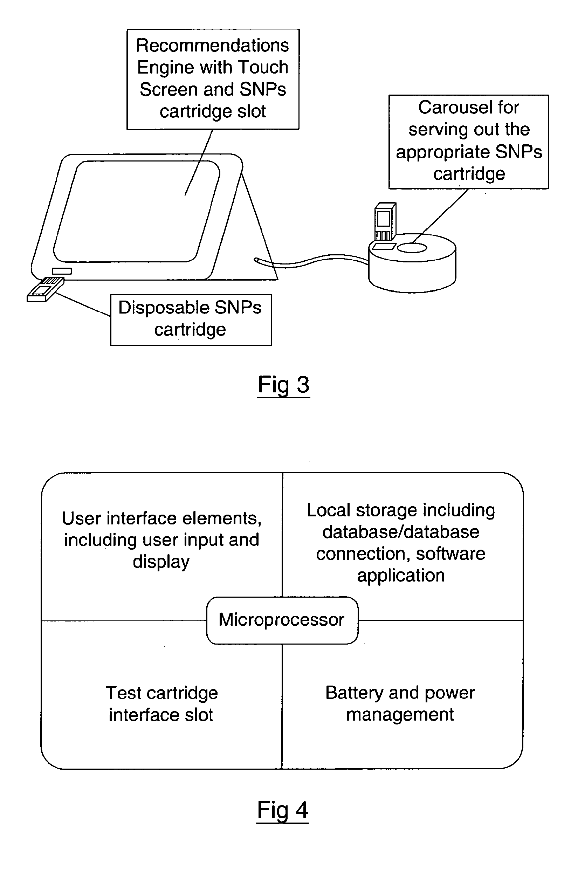

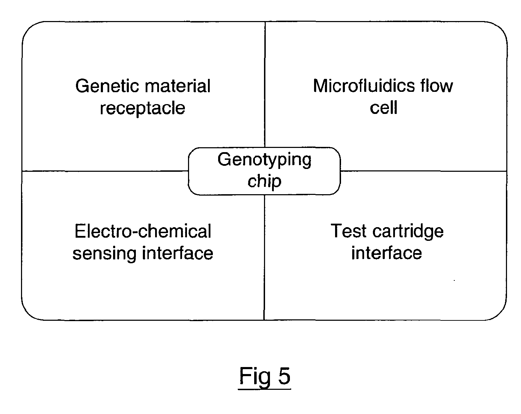

In one embodiment, this method assumes the context of marketing and identifying the appropriate cosmetic product that is suitable for a potential customer in a retail store using custom designed software and hardware device package (the "Recommendations Engine" (RE)). The RE contains a slot for interfacing with the test cartridge (TC), as shown in Figure 3. The key functional blocks that make up the RE and TC are depicted in Figure 4 and Figure 5 respectively. There will typically be a number of different TCs available, with each different TC testing a different selection of SNPs. The TCs will typically be single use and/or disposable cartridges.

Referring again to Figure 2, in Step 1, the consumer is asked to provide feedback on the type and nature of skin care product of interest (for example an anti-aging mask or a skin-whitening cream). Within a brick-and-mortar retail context, the number of cosmetic products available in a retail store is likely to be limited and within that range of available products, the cosmetic products that have associated genetic suitability test for them may encompass a subset of all the cosmetic products that have associated genetic suitability tests. The point of sale locale is expected to a be a shop or retail kiosk.

In Step 2, as there are many SNPs and arrays of SNPs that are known and yet to be discovered that may be relevant in guiding the choice of cosmetic products, and the limitation in the number of SNPs that can be genotyped at one time within a rapid-testing, portable test environment (i.e. the RE), an algorithm is used to suggest the appropriate selection of SNPs to be tested (i.e. the algorithm is used to determine which TC to use). The algorithm makes the selection from the comprehensive set of SNPs tests possible based on the consumer's indicated desires in Step 1, as well as the types of TCs available in the retail store and the selection of available skin-care products in the said retail store. Each TC is coded with a serial number, which could comprise a string of symbols such as an alphanumeric string of which a portion would be indicative of the type of SNPs being tested for in the TC. Based on the said indicative code segment, the traits and properties of the SNPs tested in the TC could be looked up within the TC catalogue database containing information on all possible SNPs and SNP configurations that are available globally. In an advanced embodiment, the selection algorithm may also be influenced by a host of other factors, including but not limited to the previous tests that have been performed (so as to offer a new set of products), the products available on promotion, and the popular products that other customers with similar traits have bought and found useful.

In Step 3, the customer's genetic material, such as from a cheek swap or saliva sample, is obtained and deposited in contact with the chemically active sensor on the selected genetic TC. The genetic material contains a polynucleotide sample comprising DNA, RNA or a combination of both.

In Step 4, the TC is inserted into the RE and an identification and configuration handshake exchange protocol is performed between the RE and TC. Within this handshaking protocol, the type of TC is communicated to the RE through the appropriate TC serial number code segment. Once this handshake is completed, rapid SNPs genotyping is performed in the TC and the results are transmitted electrically to the RE within minutes of the sample being deposited in the TC. The TC will be configured using a polynucleotide probe to perform a targeted test for the presence of one or more specific SNPs.

In Step 5, the results from the genotyping are received by the RE from the TC and translated into specific parameters, each parameter comprising a tuple of the SNP and the result of the SNP test, by querying the TC catalogue database with the relevant TC serial number code segment. An algorithm is then used to map the parameters into characteristic traits and a corresponding score for that particular trait so as to provide an output of a set of tuples of characteristic traits and their corresponding values. The output is referred to as the characteristic traits table. For each type of TC, there would be a characteristic trait table with values derived from the detected genotype of the individual.

In Step 6, the characteristic traits table is used to structure a query in an appropriate database query language (such as SQL) which is then applied to the cosmetic products database to select from the list of cosmetic products available in the retail store the subset list of products to be recommended, based on the customer's genotype information as well as the desired cosmetic product range indicated in Step 1. The query may be submitted to a local database contained within the RE or to a remote database. By transforming the SNPs responses into the characteristics trait table which is then used as filter values for the database query, it is possible to offload the database of all possible cosmetic products to a remote server and perform queries at the remote server without transmitting information that can be used to identify the said individual's genetic makeup. This is desirable as it ensures that there is a high-level of confidence that no individual genetic information is leaked from the system, without having to resort to complex encryption schemes. Onerous warranties from the service provider to the customer regarding the data integrity and security of the customer's genetic information are therefore also not required.

In Step 7, the selected list of recommended products is displayed on the RE and presented to the customer. In an advanced embodiment, a recommendation score that is proportional to the suitability of the product to the individual is displayed against each of the recommended products list. The list may then be sorted according to different parameters, including but not limited to the prices of the recommended products, their brands and their suitability score.

In Step 8, the TC may be removed from the RE and discarded, while "housekeeping" tasks are performed by the RE to update the customer database as well as to reset itself for the next customer/test.

The performance of the genetic test is wholly carried out in situ within the portable device without a need to send the consumer's genetic sample to a remote laboratory. Furthermore, as the test cartridge is single used and disposed of after the test, this provides strong assurance to the consumer that there is no risk of infringement of personal privacy through the storage and tracking of genetic information.

It will be appreciated by the person of skill in the art that various modifications may be made to the above described embodiments without departing from the scope of the present invention.

| Statements | Statement index | Neutralisation of reactive oxygen species p1 | Epidermal immune defense p2 | Collagen reinforcement p3 | Hydration p4 | Nourishment and oxygenation p5 | detoxification p6 | Inflammation risk a1 |
|---|---|---|---|---|---|---|---|---|
| I need to use moisturiser products regularly. | s1 | 0.1 | 0.1 | 0.9 | 0.0 | 0.6 | 0.2 | 0.1 |
| My living environment is smoke-free. | s2 | 0.7 | 0.8 | 0.0 | 0.1 | 0.3 | 0.6 | 0.1 |
| I sleep well. | s3 | 0.5 | 0.6 | 0.8 | 0.4 | 0.1 | 0.2 | 0.1 |
| I consume little alcohol. | s4 | 0.5 | 0.2 | 0.7 | 0.8 | 0.9 | 0.4 | 0.3 |
| My schedule is very hectic. | s5 | 0 | 0 | 0 | 0.1 | 0 | 0.1 | 0.1 |
| I suffer from hay fever. | s6 | 0.1 | 0.5 | 0 | 0.1 | 0.2 | 0.4 | 0.8 |

**Table 1**

| Gene | Gene index | Neutralisation of reactive oxygen species | Epidermal immune defense | Collagen reinforcement | Hydration | Nourishment and oxygenation | detoxification | Inflammation risk |
|---|---|---|---|---|---|---|---|---|
| | | p1 | p2 | p3 | p4 | p5 | p6 | a1 |
| MMP1 | g1 | 0.1 | 0.1 | 0.9 | 0.0 | 0.6 | 0.2 | 0.1 |
| SOD2 | g2 | 0.7 | 0.8 | 0.0 | 0.1 | 0.3 | 0.6 | 0.1 |
| GPX1 | g3 | 0.5 | 0.6 | 0.8 | 0.4 | 0.1 | 0.2 | 0.1 |
| EPHX | g4 | 0.5 | 0.2 | 0.7 | 0.8 | 0.9 | 0.4 | 0.1 |
| TNFa | g5 | 0 | 0 | 0 | 0.1 | 0 | 0.1 | 0.8 |

**Table 2**

| Compound | Compound index | Neutralisation of reactive oxygen species | Epidermal immune defense | Collagen reinforcement | Hydration | Nourishment and oxygenation | detoxification | Inflammation risk |
|---|---|---|---|---|---|---|---|---|
| | | p1 | p2 | p3 | p4 | p5 | p6 | a1 |
| NIA-114 | r1 | 0.8 | 0.7 | 0.4 | 0.3 | 0.7 | 0.6 | 0.1 |
| Azathioprine | r2 | 0.3 | 0.6 | 0.3 | 0.7 | 0.5 | 0.5 | 0.4 |
| aaa | r3 | 0.5 | 0.6 | 0.8 | 0.4 | 0.1 | 0.2 | 0.5 |
| bbb | r4 | 0.5 | 0.2 | 0.7 | 0.8 | 0.9 | 0.4 | 0.3 |
| ccc | r5 | 0 | 0 | 0 | 0.1 | 0 | 0.1 | 0.1 |
| ddd | r6 | 0 | 0 | 0.1 | 0 | 0 | 0 | 0.2 |

Table 3

## Claims

1. A method of using a point-of-sale terminal to select a product suited to an individual, the method being carried out at retail premises and comprising:

   • accessing a memory to identify a product category from a predefined set of product categories, each product category being associated with a set of products and, on the basis of said selected product category, select a genetic testing cartridge type from a set of available cartridge types, each cartridge type being configured to perform a genotype profiling test on a polynucleotide sample;
   • collecting a polynucleotide sample from the individual, wherein said step of collecting a polynucleotide sample from an individual is performed by the individual or a point-of-sale representative, the individual or point-of-sale representative applying the collected sample to a genetic testing cartridge of the selected type;
   • performing a genotype profiling test on said polynucleotide sample using a genetic testing cartridge of the selected genetic testing cartridge type; and
   • obtaining, via an interface coupling the point-of-sale terminal to the genetic testing cartridge, the test result and using the result of said test to select a product from within the identified product category,

   wherein said step of identifying a product category comprises identifying a product category to a point-of-sale terminal using a graphical user interface of the terminal, the point-of-sale terminal performing the steps of selecting a genetic testing cartridge type and using the results of said test to select a product from within the identified product category, the selection being displayed to a terminal user via said graphical user interface.

2. A method according to claim 1, wherein the step of using the result of said test to select a product comprises using the result of said test to select one or more metabolisable compounds from a group of known metabolisable compounds, and then selecting a product from the identified product category that contains at least one of the one or more selected metabolisable compounds.

3.  A method according to claim 2, wherein the one or more metabolisable compounds are selected by determining the efficacy of the known metabolisable compounds with respect to the metabolism pathway of the individual, and selecting the one or more with the highest efficacy.

4.  A method according to any one of the preceding claims, wherein the polynucleotide sample contains DNA, RNA or a combination of both.

5.  A method according to any preceding claim, wherein said genotype profiling test comprises one or more single-nucleotide polymorphism tests.

6.  A method according to any preceding claim, wherein said product is one of: a skin care product, a cosmetic, nutritional supplement, a food group, and an agricultural product.

7.  A method according to any one of the preceding claims, wherein said step of using the result of said test to select a product from within the identified product category comprises performing a lookup in a product database for the identified product category, using as lookup key the test result.

8.  A method according to claim 1 and comprising deleting all records of the test result from the point of sale terminal following selection of a product.

9.  A method according to any one of the preceding claims and comprising receiving a questionnaire response from said individual, and using said response in the selection of a genetic testing cartridge type and/or in the selection of a product.

10. A method according to any one of the preceding claims, wherein said genetic testing cartridge of the selected genetic testing cartridge type is configured to determine the efficacy of the associated product set.

11. A method according to any one of the preceding claims, wherein said step of selecting a product category comprises:

    using a polynucleotide sample collected from the individual;
    using a genetic testing cartridge to perform a genotype profiling test and using the result of the test to identify a predisposition of the individual to each of one or more conditions; and
    for a given one or combination of the identified predispositions, choosing a product category that can potentially treat the associated condition(s).

12. A method according to claim 11 and comprising using genetic information and customer information, obtained for the individual, in the identification of the condition predispositions and/or the choosing of the product category.

13. A method according to claim 12, wherein the genetic information of an individual is obtained using a cheek swab, a saliva sample or a blood test.

14. A method according to claim 12, wherein the customer information of the individual is obtained via a questionnaire

15. A point of sale terminal comprising:

    access to memory that may be local or remote to the terminal, in which memory there is stored a first database containing a set of product categories and a set of genetic testing cartridge types mapped to the product categories, and a second database containing, for each product category, a set of available products and a set of genotype profiling outcomes mapped to the available products;
    an interface adapted to couple the point of sale terminal to a genetic testing cartridge in order to receive test results from the cartridge;
    a graphical user interface adapted to receive from a terminal user an identification of a product category and for displaying to the user a selected product; and
    a processing entity adapted to

use the received identification of a product category to lookup in said first database to select a genetic testing cartridge type for receiving a test result via said interface,
use the received test result as a lookup in said second database to select a product, and
cause the graphical user interface to display the selected product to a user.

**Patentansprüche**

1. Verfahren zur Verwendung eines Verkaufsstellenterminals, um ein für eine Person geeignetes Produkt auszuwählen, wobei das Verfahren in Einzelhandelseinrichtungen durchgeführt wird und umfasst:

   - Zugreifen auf einen Speicher, um eine Produktkategorie aus einer vordefinierten Menge von Produktkategorien zu ermitteln, wobei jeder Produktkategorie eine Menge von Produkten zugeordnet ist, und um auf der Grundlage der ausgewählten Produktkategorie einen Gentestpatronentyp aus einer Menge von verfügbaren Patronentypen auszuwählen, wobei jeder Patronentyp dafür konfiguriert ist, einen Genotyp-Profilbestimmungstest an einer Polynukleotidprobe durchzuführen;
   - Aufnehmen einer Polynukleotidprobe von der Person, worin der Schritt des Aufnehmens einer Polynukleotidprobe von einer Person durch die Person oder durch einen Verkaufsstellenvertreter durchgeführt wird, wobei die Person oder der Verkaufsstellenvertreter die aufgenommene Probe auf eine Gentestpatrone des ausgewählten Typs aufträgt;
   - Durchführen eines Genotyp-Profilbestimmungstests an der Polynukleotidprobe unter Verwendung einer Gentestpatrone des ausgewählten Gentestpatronentyps; und
   - Erlangen des Testergebnisses mittels einer Schnittstelle, die das Verkaufsstellenterminal mit der Gentestpatrone koppelt, und Verwenden des Ergebnisses des Tests, um ein Produkt aus der ermittelten Produktkategorie auszuwählen,

   worin der Schritt des Ermittelns einer Produktkategorie umfasst: Ermitteln einer Produktkategorie für ein Verkaufsstellenterminal unter Verwendung einer grafischen Benutzerschnittstelle des Terminals, wobei das Verkaufsstellenterminal die Schritte durchführt: Auswählen eines Gentestpatronentyps und Verwenden der Ergebnisse des Tests, um ein Produkt aus der ermittelten Produktkategorie auszuwählen, wobei die Auswahl einem Terminalbenutzer mittels der grafischen Benutzerschnittstelle angezeigt wird.

2. Verfahren nach Anspruch 1, worin der Schritt des Verwendens des Ergebnisses des Tests, um ein Produkt auszuwählen, umfasst: Verwenden des Ergebnisses des Tests, um eine oder mehrere verstoffwechselbare Verbindungen aus einer Gruppe von bekannten verstoffwechselbaren Verbindungen auszuwählen, und dann Auswählen eines Produkts aus der ermittelten Produktkategorie, das mindestens eine der einen oder mehreren ausgewählten verstoffwechselbaren Verbindungen enthält.

3. Verfahren nach Anspruch 2, worin die eine oder die mehreren verstoffwechselbaren Verbindungen durch Bestimmen der Wirksamkeit der bekannten verstoffwechselbaren Verbindungen in Bezug auf den Stoffwechselweg der Person und Auswählen der einen oder mehreren mit der höchsten Wirksamkeit ausgewählt werden.

4. Verfahren nach einem der vorhergehenden Ansprüche, worin die Polynukleotidprobe DNS, RNS oder eine Kombination von beiden enthält.

5. Verfahren nach einem der vorhergehenden Ansprüche, worin der Genotyp-Profilbestimmungstest einen oder mehrere Einzelnukleotid-Polymorphismustests umfasst.

6. Verfahren nach einem der vorhergehenden Ansprüche, worin das Produkt eines von Folgendem ist: ein Hautpflegeprodukt, ein Kosmetikartikel, Nahrungsergänzungsmittel, eine Nahrungsmittelgruppe und ein landwirtschaftliches Produkt.

7. Verfahren nach einem der vorhergehenden Ansprüche, worin der Schritt des Verwendens des Ergebnisses des Tests, um ein Produkt aus der ermittelten Produktkategorie auszuwählen, umfasst: Durchführen einer Abfrage in einer Produktdatenbank nach der ermittelten Produktkategorie, wobei das Testergebnis als Abfrageschlüssel verwendet wird.

8. Verfahren nach Anspruch 1 und umfassend: Löschen aller Aufzeichnungen des Testergebnisses aus dem Verkaufsstellenterminal im Anschluss an die Auswahl eines Produkts.

9.  Verfahren nach einem der vorhergehenden Ansprüche und umfassend: Empfangen einer Fragebogenantwort von der Person und Verwenden der Antwort bei der Auswahl eines Gentestpatronentyps und/oder bei der Auswahl eines Produkts.

10.  Verfahren nach einem der vorhergehenden Ansprüche, worin die Gentestpatrone des ausgewählten Gentestpatronentyps dafür konfiguriert ist, die Wirksamkeit der zugeordneten Produktmenge zu bestimmen.

11.  Verfahren nach einem der vorhergehenden Ansprüche, worin der Schritt des Auswählens einer Produktkategorie umfasst:

Verwenden einer aufgenommenen Polynukleotidprobe von der Person;
Verwenden einer Gentestpatrone, um einen Genotyp-Profilbestimmungstest durchzuführen, und Verwenden des Ergebnisses des Tests, um eine Veranlagung der Person für jede unter einer oder mehreren Bedingungen zu ermitteln; und
für eine gegebene eine oder Kombination der ermittelten Veranlagungen erfolgendes Wählen einer Produktkategorie, welche die zugeordnete(n) Erkrankung(en) potenziell behandeln kann.

12.  Verfahren nach Anspruch 11 und umfassend: Verwenden von genetischer Information und Kundeninformation, die für die Person erlangt wurden, bei der Ermittlung der Erkrankungsveranlagungen und/oder beim Wählen der Produktkategorie.

13.  Verfahren nach Anspruch 12, worin die genetische Information einer Person unter Verwendung eines Mundschleimhautabstrichs, einer Speichelprobe oder eines Bluttests erlangt wird.

14.  Verfahren nach Anspruch 12, worin die Kundeninformation der Person mittels eines Fragebogens erlangt wird.

15.  Verkaufsstellenterminal, umfassend:

Zugriff auf Speicher, der lokal oder vom Terminal entfernt sein kann, wobei in diesem Speicher eine erste Datenbank, die eine Menge von Produktkategorien und eine Menge von Gentestpatronentypen, die den Produktkategorien zugeordnet sind, enthält, und eine zweite Datenbank, die für jede Produktkategorie eine Menge von verfügbaren Produkten und eine Menge von Genotyp-Profilbestimmungsergebnissen, die den verfügbaren Produkten zugeordnet sind, enthält, gespeichert sind;
eine Schnittstelle, die dafür eingerichtet ist, das Verkaufsstellenterminal mit einer Gentestpatrone zu koppeln, um Testergebnisse von der Patrone zu empfangen;
eine grafische Benutzerschnittstelle, die dafür eingerichtet ist, von einem Terminalbenutzer eine Kennzeichnung einer Produktkategorie zu empfangen und dem Benutzer ein ausgewähltes Produkt anzuzeigen; und
eine Verarbeitungsinstanz, die dafür eingerichtet ist:

die empfangene Kennzeichnung einer Produktkategorie zu verwenden, um in der ersten Datenbank eine Abfrage durchzuführen, um einen Gentestpatronentyp zum Empfangen eines Testergebnisses mittels der Schnittstelle auszuwählen,
das empfangene Testergebnis als eine Abfrage in der zweiten Datenbank zu verwenden, um ein Produkt auszuwählen, und
die grafische Benutzerschnittstelle zu veranlassen, einem Benutzer das ausgewählte Produkt anzuzeigen.

**Revendications**

1.  Procédé d'utilisation d'un terminal de point de vente pour choisir un produit adapté à un individu, le procédé s'effectuant dans un établissement commercial et comprenant :

• l'accès à une mémoire afin d'identifier une catégorie de produits dans un ensemble prédéfini de catégories de produits, chaque catégorie de produits étant associée à un ensemble de produits et, compte tenu de ladite catégorie de produits choisie, choisir un type de cartouche de test génétique dans un ensemble de types de cartouche disponibles, chaque type de cartouche étant configuré pour effectuer un test de profil génotypique sur un échantillon polynucléotidique ;

• le prélèvement d'un échantillon polynucléotidique chez l'individu, ladite étape consistant à prélever un échantillon polynucléotidique chez un individu étant effectué par l'individu ou un préposé du point de vente, l'individu ou le préposé du point de vente appliquant l'échantillon prélevé à une cartouche de test génétique du type choisi ;

• la réalisation d'un test de profil génotypique sur ledit échantillon polynucléotidique au moyen d'une cartouche de test génétique du type choisi de cartouche de test génétique ; et

• l'obtention, par le biais d'une interface reliant le terminal de point de vente à la cartouche de test génétique, du résultat du test et l'exploitation du résultat dudit test pour choisir un produit dans la catégorie de produits identifiée,

dans lequel ladite étape consistant à identifier une catégorie de produits comprend l'identification d'une catégorie de produits pour un terminal de point de vente au moyen d'une interface utilisateur graphique du terminal, le terminal de point de vente réalisant les étapes consistant à choisir un type de cartouche de test génétique et à exploiter les résultats dudit test pour choisir un produit dans la catégorie de produits identifiée, le choix étant présenté à un utilisateur du terminal sur ladite interface utilisateur graphique.

2. Procédé selon la revendication 1, dans lequel l'étape consistant à exploiter le résultat dudit test pour choisir un produit comprend l'exploitation du résultat dudit test pour choisir un ou plusieurs composés métabolisables dans un groupe de composés métabolisables connus, puis le choix d'un produit dans la catégorie de produits identifiée qui contient au moins un des un ou plusieurs composés métabolisables choisis.

3. Procédé selon la revendication 2, dans lequel le choix des un ou plusieurs composés métabolisables s'effectue en déterminant l'efficacité des composés métabolisables connus eu égard à la voie métabolique de l'individu, et en choisissant les un ou plusieurs composés métabolisables à l'efficacité la plus élevée.

4. Procédé selon l'une quelconque des revendications précédentes, dans lequel l'échantillon polynucléotidique contient de l'ADN, de l'ARN ou une combinaison de ceux-ci.

5. Procédé selon l'une quelconque des revendications précédentes, dans lequel ledit test de profil génotypique comprend un ou plusieurs tests de polymorphisme mononucléotidique.

6. Procédé selon l'une quelconque des revendications précédentes, dans lequel ledit produit est un produit parmi : un produit de soin de la peau, un produit cosmétique, un supplément alimentaire, un groupe alimentaire et un produit agricole.

7. Procédé selon l'une quelconque des revendications précédentes, dans lequel ladite étape consistant à exploiter le résultat dudit test pour choisir un produit dans la catégorie de produits identifiée comprend la réalisation d'une consultation dans une base de données de produits relativement à la catégorie de produits identifiée, en employant le résultat du test comme clé de consultation.

8. Procédé selon la revendication 1 et comprenant la suppression de toute trace du résultat du test sur le terminal de point de vente à la suite du choix d'un produit.

9. Procédé selon l'une quelconque des revendications précédentes et comprenant la réception d'une réponse à un questionnaire fournie par l'individu, et l'exploitation de ladite réponse dans le choix d'un type de cartouche de test génétique et/ou dans le choix d'un produit.

10. Procédé selon l'une quelconque des revendications précédentes, dans lequel ladite cartouche de test génétique du type choisi de cartouche de test génétique est configurée pour déterminer l'efficacité de l'ensemble de produits associé.

11. Procédé selon l'une quelconque des revendications précédentes, dans lequel ladite étape consistant à choisir une catégorie de produits comprend :

l'utilisation d'un échantillon polynucléotidique prélevé chez l'individu ;
l'emploi d'une cartouche de test génétique pour réaliser un test de profil génotypique et l'exploitation du résultat du test pour identifier une prédisposition de l'individu à chacune parmi une ou plusieurs pathologies ; et
pour une prédisposition donnée ou la combinaison des prédispositions identifiées, le choix d'une catégorie de produits susceptible de traiter la ou les pathologies associées.

12. Procédé selon la revendication 11 et comprenant l'exploitation d'informations génétiques et d'informations clients, obtenues relativement à l'individu, dans l'identification des prédispositions à la pathologie et/ou dans le choix de la catégorie de produits.

13. Procédé selon la revendication 12, dans lequel les informations génétiques d'un individu sont obtenues au moyen d'un frottis de la joue, d'un échantillon de salive ou d'un test sanguin.

14. Procédé selon la revendication 12, dans lequel les informations clients relatives à l'individu sont obtenues au moyen d'un questionnaire.

15. Terminal de point de vente comprenant :

l'accès à une mémoire, qui peut être locale ou distante du terminal, renfermant une première base de données contenant un ensemble de catégories de produits et un ensemble de types de cartouche de test génétique mis en correspondance avec les catégories de produits, et une deuxième base de données contenant, pour chaque catégorie de produits, un ensemble de produits disponibles et un ensemble de résultats de profil génotypique mis en correspondance avec les produits disponibles ;
une interface adaptée pour relier le terminal de point de vente à une cartouche de test génétique dans le but de recevoir les résultats de test en provenance de la cartouche ;
une interface utilisateur graphique adaptée pour recevoir, de la part d'un utilisateur du terminal, l'identification d'une catégorie de produits, et pour présenter à l'utilisateur un produit choisi ; et
une entité de traitement adaptée pour :

utiliser la réception de l'identification d'une catégorie de produits pour consulter ladite première base de données afin de choisir un type de cartouche de test génétique, en vue de recevoir un résultat de test par le biais de ladite interface,
exploiter le résultat de test reçu comme correspondance dans ladite deuxième base de données pour choisir un produit, et
amener l'interface utilisateur graphique à afficher le produit choisi à l'attention d'un utilisateur.

<u>Fig 1</u>

```
┌─────────────────────────────────────────┐
│                0. Start                  │
└─────────────────────────────────────────┘
                    ⇩
┌─────────────────────────────────────────┐
│        1. Selection of skin care         │
│              product type                │
└─────────────────────────────────────────┘
                    ⇩
┌─────────────────────────────────────────┐
│        2. Selection of genetic           │
│             test cartridge               │
└─────────────────────────────────────────┘
                    ⇩
┌─────────────────────────────────────────┐
│       3. Genetic material sampling       │
└─────────────────────────────────────────┘
                    ⇩
┌─────────────────────────────────────────┐
│         4. Rapid SNPs Genotyping         │
└─────────────────────────────────────────┘
                    ⇩
┌─────────────────────────────────────────┐
│       5. Extract individual traits and   │
│          sensitivities from genotype     │
└─────────────────────────────────────────┘
                    ⇩
┌─────────────────────────────────────────┐
│    6. Generate query to skin care products│
│       database based on individual traits.│
└─────────────────────────────────────────┘
                    ⇩
┌─────────────────────────────────────────┐
│ 7. Presentation of list of suitable products and│
│  its potential responsiveness to the individual │
└─────────────────────────────────────────┘
                    ⇩
┌─────────────────────────────────────────┐
│ 8. Test cartridge may be discarded and the│
│      customer database is updated         │
└─────────────────────────────────────────┘
```

Fig 2

Recommendations
Engine with Touch
Screen and SNPs
cartridge slot

Carousel for
serving out the
appropriate SNPs
cartridge

Disposable SNPs
cartridge

## Fig 3

User interface elements,
including user input and
display

Local storage including
database/database
connection, software
application

Microprocessor

Test cartridge
interface slot

Battery and power
management

## Fig 4

| Genetic material receptacle | Microfluidics flow cell |
| --- | --- |
| Electro-chemical sensing interface | Test cartridge interface |

Genotyping chip

## Fig 5

**REFERENCES CITED IN THE DESCRIPTION**

**Patent documents cited in the description**

- GB 2389424 A